# EUROPEAN PATENT APPLICATION

(11) **EP 4 541 413 A1**
(43) Date of publication of application: **23.04.2025**
(21) Application number: 23829881.4
(22) Date of filing: 06.06.2023
(51) Int. Cl.: A61N 1/05

(54) **CRANIAL HOLE ELECTRODE SECURING DEVICE**

(30) Priority: 29.06.2022 CN 202210752478
(71) Applicant: SceneRay Co., Ltd., Jiangsu 215123 (CN)
(72) Inventor: YAN, Hao, Suzhou, Jiangsu 215123 (CN); WU, Guoliang, Suzhou, Jiangsu 215123 (CN); ZHU, Weiran, Suzhou, Jiangsu 215123 (CN)
(74) Representative: Cabinet Beaumont
(86) International application number: PCT/CN2023/098648
(87) International publication number: WO 2024/001697

(57) **Abstract**

Provided is a cranial hole electrode securing device. A cranial ring is secured on a cranium. The cranial ring includes a ring portion and an extension portion. The extension portion is connected to the bottom end of the ring portion, and the cranial ring is provided with a through hole. At least part of the electrode lock is disposed in the through hole of the extension portion. The electrode lock is provided with a clamping opening. The locking assembly is disposed at the bottom end of the ring portion and sleeved on the outer side of the extension portion. When the locking assembly is moved from a first position to a second position, the locking assembly applies an acting force to the extension portion, and an opening at the bottom end of the through hole contracts and compresses the electrode lock, causing a positional lock of the electrode lock and/or causing the electrode to be clamped. When the locking assembly is moved from the second position to the first position, the acting force applied by the locking assembly on the extension portion disappears or decreases, the opening at the bottom end of the through hole does not compress the electrode lock, and the position of the electrode lock is unlocked or the electrode is released from the constraint of the electrode lock. Thus, the displacement of the electrode lock is reduced, thereby reducing electrode stimulation failures and ensuring the effectiveness of electrode stimulation.

## Description

This application claims priority to Chinese Patent Application No. CN202210752478.4 filed on Jun. 29, 2022, which is incorporated herein by reference in its entirety.

### TECHNICAL FIELD

The present application relates to the technical field of medical devices, for example, a cranial hole electrode securing device.

### BACKGROUND

Implantable medical systems typically include an implantable nerve stimulation system, an implantable cardiac stimulation system (known as a cardiac pacemaker), and an implantable medication infusion system. The implantable nerve stimulation system includes a deep brain stimulation, an implantable cortical nerve stimulation, an implantable spinal cord stimulation, an implantable sacral nerve stimulation, and an implantable vagus nerve stimulation. For example, the implantable nerve stimulation system mainly includes a pulse generator and an electrode implanted inside a body, and a control device outside the body. The pulse generator is connected to the electrode such that a pulse generated by the pulse generator is transmitted to the electrode. A pulse signal generated by the pulse generator is transmitted to a brain or particular nerve sites by the electrode and restores normal functionalities of the body through electrical stimulation.

A surgeon first determines the position of a target in the brain of a patient through a stereotaxic device during an implantation operation. Then, the surgeon drills a small hole in the patient's cranium according to the position of the target and inserts the electrode into the target in the brain through the cranial hole. The surgeon uses a test stimulator to perform a test on the target. If the effect of stimulation therapy is desirable, the electrode is caused to penetrate through an electrode lock and be secured on the cranium. The electrode lock is configured to clamp the electrode. However, since the stimulated point in the brain is often very small, any slight displacement of the electrode used for brain stimulation may cause the stimulation to fail.

Therefore, there is an urgent need for a cranial hole electrode securing device that can reduce the displacement of the electrode lock, thereby reducing electrode stimulation failures and ensuring the effectiveness of electrode stimulation.

### SUMMARY

An object of the present application is to provide a cranial hole electrode securing device configured to reduce the displacement of an electrode lock, thereby reducing electrode stimulation failures and ensuring the effectiveness of electrode stimulation.

The object of the present application is achieved by the technical solutions below.

The present application provides a cranial hole electrode securing device. The cranial hole electrode securing device includes a cranial ring, an electrode lock, and a locking assembly.

The cranial ring is configured to be secured on a cranium, where the cranial ring includes a ring portion and an extension portion, the extension portion is connected to the bottom end of the ring portion, and the cranial ring is provided with a through hole penetrating through the ring portion and the extension portion.

The electrode lock is configured to clamp an electrode, where at least part of the electrode lock is disposed in the through hole of the extension portion, and the electrode lock is provided with a clamping opening configured for the electrode to penetrate through.

The locking assembly is movably disposed at the bottom end of the ring portion and is sleeved on the outer side of the extension portion. When the locking assembly is moved from a first position to a second position, the locking assembly applies an acting force to the extension portion, and an opening at the bottom end of the through hole contracts and compresses the electrode lock, causing a positional lock of the electrode lock and/or causing the clamping opening to contract to clamp the electrode. When the locking assembly is moved from the second position to the first position, the acting force applied by the locking assembly on the extension portion disappears or decreases, the opening at the bottom end of the through hole does not compress the electrode lock, causing the positional lock of the electrode lock to be released or causing the constraint of the electrode lock on the electrode to be released.

In a possible implementation, a sidewall of the extension portion is provided with at least one contraction groove extending upward from the bottom of the extension portion and penetrating through the inside and the outside of the extension portion, the extension portion is evenly divided into at least two connected parts by the at least one contraction groove, the outer sidewall of each of at least one part of the extension portion is provided with at least one first protrusion, and an inner sidewall of the locking assembly is provided with at least one second protrusion corresponding in position to the at least one first protrusion.

When the locking assembly is rotated such that each first protrusion abuts against a the respective second protrusion, the locking assembly applies the acting force on each first protrusion of the extension portion through a respective second protrusion, parts of the extension portion with the at least one first protrusion converge toward the central axis of the extension portion and compress the electrode lock to contract the clamping opening to clamp the electrode.

When the locking assembly is rotated such that each first protrusion is away from a respective second protrusion, each part of the extension portion does not compress the electrode lock.

In a possible implementation, the ring portion is provided with at least one lug slot, the locking assembly includes a sleeve and at least one extension lug, the at least one extension lug is connected to the edge of the top of the sleeve and extends away from the central axis of the sleeve, the at least one extension lug corresponds in position to matches in size with the at least one lug slot, and the bottom of each of the at least one extension lug is configured to be in contact with the cranium.

When the sleeve is rotated from the first position to the second position, each of the at least one extension lug is rotated from one side of a respective lug slot to the other side of the respective lug slot, each of the at least one first protrusion is caused to abut against a respective second protrusion, and parts of the extension portion with the at least one first protrusion converge toward the central axis of the extension portion to compress the electrode lock to clamp the electrode.

In a possible implementation, the cranial hole electrode securing device further includes a cranial hole cover, where the ring portion is provided with at least one snapping slot, and the cranial hole cover is engaged with the cranial ring through the at least one snapping slot and is configured to seal the electrode lock in the through hole.

When the cranial hole cover snaps into the at least one snapping slot, the top surface of the cranial hole cover is not higher than the top surface of the ring portion.

In a possible implementation, the locking assembly further includes limiting columns, where each of the limiting columns is connected to the side of a respective extension lug facing the cranial hole cover, and the cranial hole cover is provided with limiting holes which correspond in position to and match in size with the limiting columns.

When the cranial hole cover is connected to the cranial ring, each of the limiting columns is inserted into a respective one of the limiting holes and restrains the sleeve from rotating from the second position to the first position.

In a possible implementation, the outer diameter of the sleeve is not greater than 8 mm, the height of the sleeve is not greater than the depth of the cranial hole, the ring portion is configured to fit snugly against the outer surface of the cranium, and the height of the ring portion is not greater than 2 mm.

In a possible implementation, the inner wall of the through hole in the extension portion is provided with a ring-shaped slot structure extending outward and transitioning smoothly to the rest part of the inner wall of the through hole, the part of the electrode lock located in the through hole of the extension portion has a universal ball joint structure which snugly fits against the inner wall of the through hole in the extension portion, the part of the electrode lock extending below the through hole tapers from top to bottom, and the size of an opening at the lower end of the clamping opening is not greater than the size of an opening at the upper end of the clamping opening.

In a possible implementation, the electrode lock includes a tightening portion and a clamping portion that communicate with each other, the tightening portion and the clamping portion are integrally connected to each other, the tightening portion is at least partially disposed in the through hole of the extension portion, and a lower-end part of the clamping portion is located outside and below the through hole.

In a possible implementation, the material of the electrode lock is silica gel, the tightening portion has a universal ball joint structure, and the clamping portion is a conical frustum structure whose large end is connected to the tightening portion.

In a possible implementation, the material of the electrode lock is plastic, and the tightening portion and the clamping portion are integrally connected to each other to form a collet structure.

In a possible implementation, the material of the locking assembly is metal.

In a possible implementation, the material of the locking assembly is a titanium alloy or stainless steel.

In a possible implementation, the ring portion is provided with at least two threaded holes, and each of the at least two threaded holes is configured to be penetrated through by a screw to secure the cranial ring on the cranium.

In a possible implementation, the ring portion is provided with multiple securing slots configured to secure the electrode, and the multiple securing slots are evenly distributed about the central axis of the cranial ring.

The cranial hole electrode securing device provided by the present application has at least the advantages below.

During an operation, a cranial hole is first made on the cranium of a patient. The extension portion of the cranial ring is caused to correspond to the cranial hole and the cranial ring is secured on the cranium. The electrode is inserted into the clamping opening and caused to correspond to a target in the brain. Then, when being moved from the first position to the second position, the locking assembly applies the acting force to the extension portion and causes the opening at the bottom end of the through hole to contract and compress the electrode lock so that the position of the electrode lock is locked and the electrode is clamped. Thus, with the cranial hole electrode securing device, electrode stimulation failures are reduced and the effectiveness of electrode stimulation is ensured.

### BRIEF DESCRIPTION OF DRAWINGS

The present application is further described below in conjunction with drawings and embodiments.
FIG. 1 is structural view one of a cranial hole electrode securing device according to an embodiment of the present application;
FIG. 2 is structural view two of a cranial hole electrode securing device according to an embodiment of the present application;
FIG. 3 is a structural view of a cranial ring in a cranial hole electrode securing device according to an embodiment of the present application;
FIG. 4 is a top view of a cranial ring in a cranial hole electrode securing device according to an embodiment of the present application;
FIG. 5 is a structural view showing that a cranial ring mates with a locking assembly in a cranial hole electrode securing device according to an embodiment of the present application;
FIG. 6 is a structural view showing that a cranial ring mates with a first electrode lock in a cranial hole electrode securing device according to an embodiment of the present application;
FIG. 7 is a structural view showing that a cranial ring mates with a second electrode lock in a cranial hole electrode securing device according to an embodiment of the present application;
FIG. 8 is a structural view of a cranial hole cover in a cranial hole electrode securing device according to an embodiment of the present application;
FIG. 9 is a structural view of a sleeve in a cranial hole electrode securing device according to an embodiment of the present application;
FIG. 10 is a structural view of a first electrode lock in a cranial hole electrode securing device according to an embodiment of the present application; and
FIG. 11 is a structural view of a second electrode lock in a cranial hole electrode securing device according to an embodiment of the present application.

### Reference list:

- 1: cranial ring
- 2: ring portion
- 3: extension portion
- 4: through hole
- 5: electrode lock
- 500: tightening portion
- 501: clamping portion
- 6: clamping opening
- 7: locking assembly
- 10: contraction groove
- 11: first protrusion
- 12: second protrusion
- 13: lug slot
- 14: sleeve
- 15: extension lug
- 16: cranial hole cover
- 17: snapping slot
- 18: limiting column
- 19: limiting hole
- 20: threaded hole
- 21: securing slot

### DETAILED DESCRIPTION

Example embodiments are described more fully with reference to the drawings. However, the example embodiments may be implemented in many different forms and should not be construed as limited to the embodiments set forth herein. Rather, these embodiments are provided so that the present invention is thorough and complete and fully conveys the concept of the example embodiments to those skilled in the art. The same reference numerals in the drawings denote the same or similar structures, and thus the description of the same reference numerals is not repeated.

Expressions of positions and directions in the present application are described by using the drawings as an example. However, changes may be made based on a requirement, and the changes fall within the scope of the present application.

The present application provides a cranial hole electrode securing device with a cranial ring 1, an electrode lock 5, and a locking assembly 7.

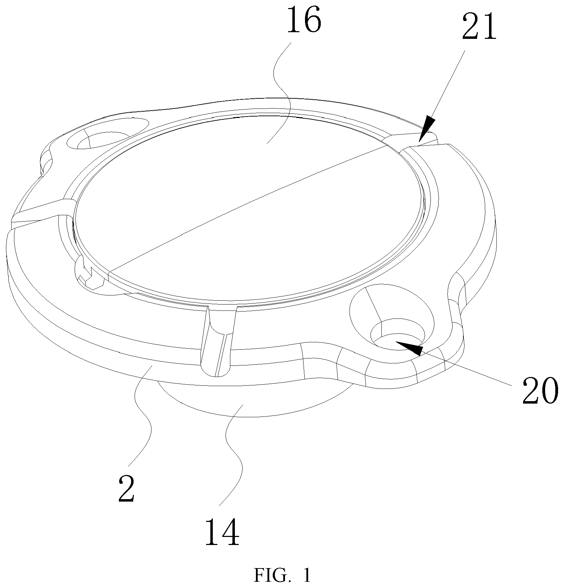

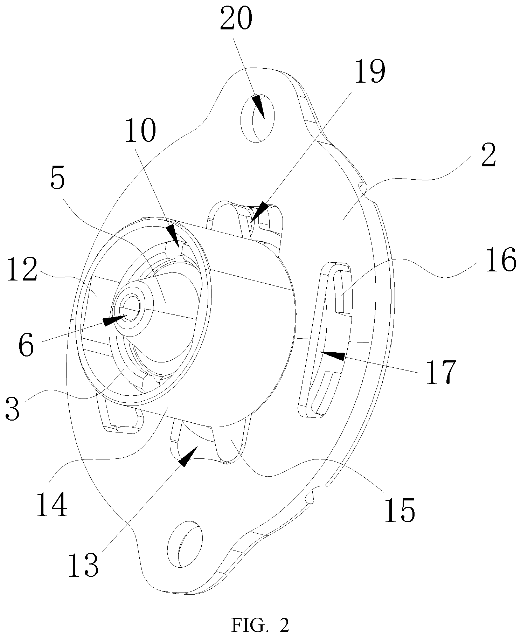

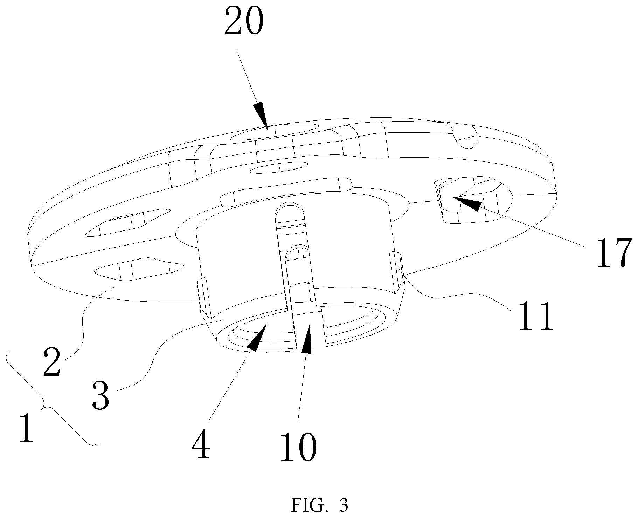

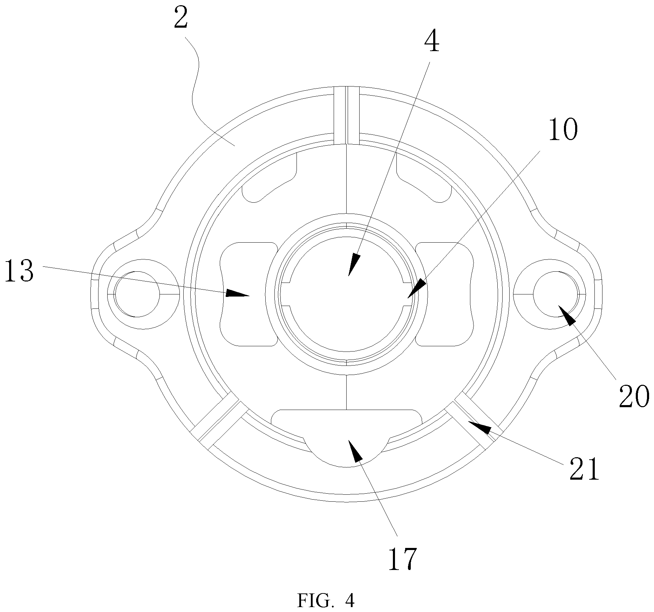

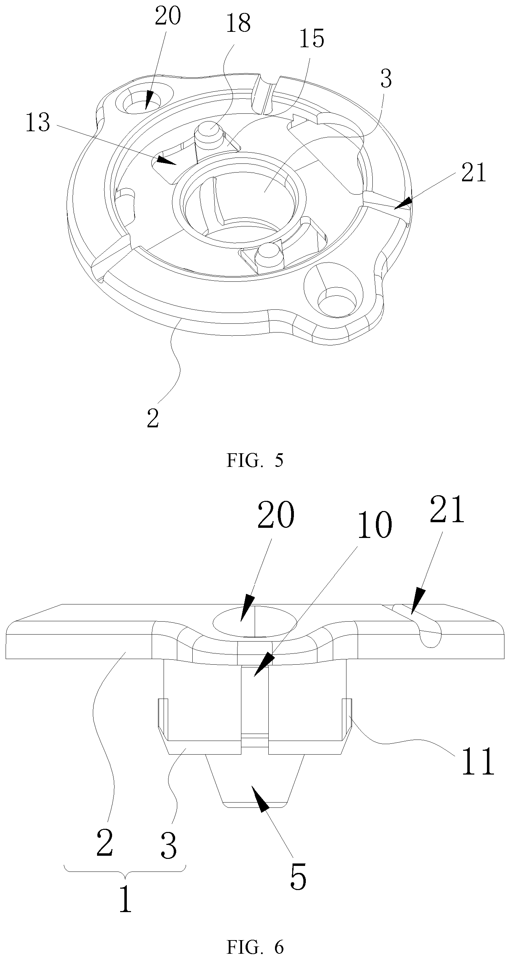

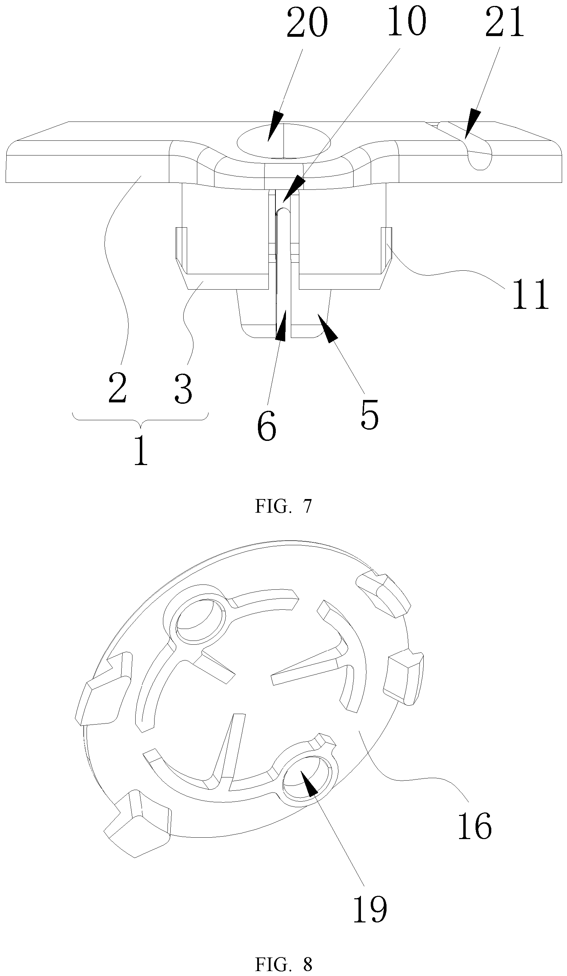

As shown in FIGS. 1 to 7, FIG. 1 is structural view one of the cranial hole electrode securing device according to an embodiment of an application of the present invention, FIG. 2 is structural view two of the cranial hole electrode securing device according to an embodiment of the application of the present invention, FIG. 3 is a structural view of the cranial ring in the cranial hole electrode securing device according to an embodiment of the application of the present invention, FIG. 4 is a top view of the cranial ring in the cranial hole electrode securing device according to an embodiment of the application of the present invention, FIG. 5 is a structural view showing that the cranial ring mates with the locking assembly in the cranial hole electrode securing device according to an embodiment of the application of the present invention, FIG. 6 is a structural view showing that the cranial ring mates with a first electrode lock in the cranial hole electrode securing device according to an embodiment of the application of the present invention, and FIG. 7 is a structural view showing that the cranial ring mates with a second electrode lock in the cranial hole electrode securing device according to an embodiment of the application of the present invention. The cranial ring 1 is configured to be secured on the cranium of a patient. The cranial ring 1 includes a ring portion 2 and an extension portion 3. The extension portion 3 is connected to the bottom end of the ring portion 2. The cranial ring 1 is provided with a through hole 4 penetrating through the ring portion 2 and the extension portion 3. The ring portion 2 is provided with two threaded holes 20. The two threaded holes 20 are penetrated through by two screws to secure the cranial ring 1 on the brain. The threaded holes 20 are distributed on two sides of the ring portion 2, thereby preventing the cranial ring 1 from warping. The electrode lock 5 is configured to clamp an electrode. At least part of the electrode lock 5 is disposed in the through hole 4 of the extension portion 3. The electrode lock 5 is provided with a clamping opening 6 configured for the electrode to penetrate through. The locking assembly 7 is movably disposed at the bottom end of the ring portion 2 and is sleeved on the outer side of the extension portion 3. When the locking assembly 7 is moved from a first position to a second position, the locking assembly 7 applies an acting force to the extension portion 3, and an opening at the bottom end of the through hole 4 contracts and compresses the electrode lock 5, causing a positional lock of the electrode lock 5 so that the electrode lock 5 is unmovable. At the same time, the clamping opening 6 of the electrode lock 5 further contracts and clamps the electrode. When the locking assembly 7 is moved from the second position to the first position, the acting force applied by the locking assembly 7 on the extension portion 3 disappears or decreases, and the opening at the bottom end of the through hole 4 does not compress the electrode lock 5. In this case, the position of the electrode lock 5 is unlocked and the electrode lock 5 is movable in the through hole 4. Thus, the electrode lock 5 is adaptable to more electrode implantation angles, and the clamping force of the electrode lock 5 on the electrode is reduced or the electrode is completely released from the constraint of the electrode lock 5.

In some possible implementations, when a medical personnel performs an operation, a cranial hole is first made on the cranium of the patient. Then, the extension portion 3 and the locking assembly 7 are caused to correspond to the cranial hole, and the two screws respectively penetrate through the two threaded holes 20 to secure the cranial ring 1 to the outer side of the cranium. In this case, the extension portion 3 and the locking assembly 7 are at least partially located in the cranial hole. Then, the electrode is caused to penetrate through the clamping opening 6 and correspond to a target in the brain. Finally, when the locking assembly 7 is moved from the first position to the second position, the locking assembly 7 applies the acting force to the extension portion 3, and the opening at the bottom end of the through hole 4 contracts and compresses the electrode lock 5, causing a positional lock of the electrode lock 5 so that the electrode lock 5 does not move. Moreover, during the compression, the clamping opening 6 of the electrode lock further contracts and clamps the electrode. When the electrode needs to be adjusted or the cranial hole electrode securing device needs to be removed, the locking assembly 7 is moved from the second position to the first position. In this case, the acting force applied by the locking assembly 7 on the extension portion 3 disappears or decreases, and the opening at the bottom end of the through hole 4 does not compress the electrode lock 5. Thus, the position of the electrode lock 5 is unlocked and the electrode lock 5 is movable in the through hole. The clamping force of the electrode lock 5 on the electrode is reduced or the electrode is completely released from the constraint of the electrode lock 5. This structure can effectively avoid the displacement of the electrode lock 5 after the electrode is clamped, thereby reducing the possibility of an electrode stimulation failure and ensuring the effectiveness of electrode stimulation.

In some possible implementations, as shown in FIGS. 3, 6, 7, and 9, FIG. 9 is a structural view of a sleeve in the cranial hole electrode securing device according to an embodiment of the application of the present invention. A sidewall of the extension portion 3 is provided with at least one contraction groove 10 extending upward from the bottom of the extension portion 3 and penetrating through the inside and the outside of the extension portion 3. The extension portion 3 is evenly divided into at least two connected parts by the at least one contraction groove 10. In some possible implementations, the extension portion 3 is evenly divided into the at least two connected parts by the at least one contraction groove 10. Since the locking assembly 7 applies the acting force to the extension portion 3 when the locking assembly 7 is moved from the second position to the first position, the at least one contraction groove 10 is provided so that when the acting force is applied to the extension portion 3, each part of the extension portion 3 can converge toward the central axis of the through hole 4. Thus, the extension portion 3 and the locking assembly 7 are effectively prevented from rigidly squeezing each other to be damaged. Thus, the service life of the cranial hole electrode securing device is prolonged. One first protrusion 11 is disposed on the outer sidewall of each part of the extension portion 3, and the inner sidewall of the locking assembly 7 is provided with a second protrusion 12 corresponding in position to the first protrusion. In some possible implementations, when the locking assembly 7 is rotated such that the second protrusion 12 abuts against the first protrusion 11, the locking assembly 7 applies the acting force to the first protrusion 11 of the extension portion 3 through the second protrusion 12. Parts of the extension portion 3 with first protrusions 11 converge toward the central axis of the extension portion 3 and compress the electrode lock 5 to lock the position of the electrode lock and contract the clamping opening 6 to clamp the electrode. In this case, the position of the locking assembly 7 may be defined as the first position. In addition, the surface of the first protrusion 11 facing the second protrusion 12 is an arc surface and the surface of the second protrusion 12 facing the first protrusion 11 is a plane so that the locking assembly 7 is rotated more smoothly and the difficulty of the operation is reduced. When the locking assembly 7 is rotated such that the second protrusion 12 is away from the first protrusion 11, each part of the extension portion 3 does not compress the electrode lock 5. In this case, the position of the locking assembly 7 may be defined as the second position. With the preceding cranial hole electrode securing device, the locking assembly 7 is rotated so that the electrode can be clamped, thereby greatly simplifying a surgical procedure and reducing the difficulty of the operation.

In some possible implementations, as shown in FIGS. 2, 4, 5, and 9, the ring portion 2 is provided with at least one lug slot 13, and the locking assembly 7 includes a sleeve 14 and at least one extension lug 15. In some possible implementations, two extension lugs 15 are provided. The two extension lugs 15 are connected to the left and right edges of the top of the sleeve 14 respectively and extend away from the central axis of the sleeve 14. The position of each of the two extension lugs 15 corresponds to the position of a respective one of the two lug slots 13, and the size of each of the two extension lugs 15 is matched with the size of the respective one of the two lug slots 13. In some possible implementations, during the operation, the locking assembly 7 and the extension portion 3 of the cranial ring 1 are correspondingly inserted into the premade cranial hole. Then, the cranial ring is secured on the cranium through the two screws. It is to be noted here that the bottoms of the extension lugs 15 are in contact with the outer side of the cranium in this case. When the sleeve 14 is rotated from the first position to the second position, the sleeve 14 is at the second position in this case, and each of the two extension lugs 15 moves from one side of the respective one of the two lug slots 13 to the other side of the respective one of the two lug slots 13, as shown in FIG. 5. In this case, the second protrusion 12 abuts against the first protrusion 11, and the parts of the extension portion 3 with the first protrusions 11 converge toward the central axis of the extension portion 3 and compress the electrode lock 5 to clamp the electrode. Then, the position of the electrode can be locked. The two extension lugs 15 are provided so that the locking assembly 7 can be prevented from falling into the brain via the cranial hole to cause a medical accident. In addition, it is convenient for the locking assembly 7 to rotate in the sleeve 14, the surgical procedure is simplified, and the difficulty of the operation is reduced. In some possible implementations, when the sleeve 14 is rotated, the medical personnel can directly rotate the extension lugs 15 manually to drive the sleeve 14 to rotate from the first position to the second position. Alternatively, the medical personnel can rotate the extension lugs 15 with a special tool to drive the sleeve 14 to rotate from the first position to the second position.

In some possible implementations, as shown in FIGS. 1 and 2, the cranial hole electrode securing device further includes a cranial hole cover 16. The ring portion 2 is provided with at least one snapping slot 17. After the electrode lock 5 completes securing the electrode, the cranial hole cover 16 is caused to snap into the cranial ring 1 through the at least one snapping slot 17 and is configured to seal the electrode lock 5 in the through hole 4, thereby not only facilitating assembly but also reducing the difficulty of the operation. Moreover, to prevent the cranial hole electrode securing device from being excessively protrusive to affect the appearance of the patient's head, the top surface of the cranial hole cover 16 is not higher than the top surface of the ring portion 2 when the cranial hole cover 16 snaps into the at least one snapping slot 17.

In some possible implementations, as shown in FIGS. 2, 5, 8, and 9, the locking assembly 7 further includes limiting columns 18. Each of the limiting columns 18 is connected to the side of a respective one of the extension lugs 15 facing the cranial hole cover 16. The cranial hole cover 16 is provided with limiting holes 19 whose positions correspond to the positions of the limiting columns 18 and whose sizes are matched with the sizes of the limiting columns 18. After the electrode lock 5 completes securing the electrode, the cranial hole cover 16 can be connected to the cranial ring 1. In this case, each of the limiting columns 18 is inserted into the respective one of the limiting holes 19, and the sleeve 14 cannot rotate from the second position to the first position. Thus, the case is avoided where the electrode lock 5 has no constraint on the electrode. The preceding structure is not only simple but also has a foolproof function, effectively ensuring the reliability of the cranial hole electrode securing device.

In some possible implementations, to reduce the size of the cranial hole, the outer diameter of the sleeve 14 is not greater than 8 mm, the height of the sleeve 14 is not greater than the depth of the cranial hole, the ring portion 2 fits snugly against the outer surface of the cranium, and the height of the ring portion 2 is not greater than 2 mm. The preceding structure can not only reduce the size of the cranial hole but also prevent the cranial hole electrode securing device from being excessively inserted into the brain, thus avoiding an inflammation and the like.

In some possible implementations, as shown in FIGS. 5 to 7, the inner wall of the through hole 4 in the extension portion 3 has a ring-shaped slot structure extending outward and transitioning smoothly to the rest part of the inner wall of the through hole 4, and the part of the electrode lock 5 located in the through hole 4 of the extension portion 3 has a universal ball joint structure which fits snugly against the inner wall of the through hole 4 in the extension portion 3. Thus, when the electrode is obliquely inserted into the clamping opening 6, the electrode lock 5 is rotatable to a certain angle to cope with the case where the electrode is displaced due to the deviation of an electrode implantation angle. In addition, a more flexible electrode implantation path plan is provided to adapt to more electrode implantation angles. Moreover, the part of the electrode lock 5 extending outside and below the through hole 4 tapers from top to bottom. This structure reduces the size and cost of the electrode lock 5. In addition, the size of an opening at the lower end of the clamping opening 6 is not greater than the size of an opening at the upper end of the clamping opening 6. Thus, the opening at the upper end of the clamping opening 6 is configured to be relatively large so that the electrode is conveniently inserted into the clamping opening 6, thereby reducing the difficulty of the operation, and the opening at the lower end of the clamping opening 6 is configured to be relatively small so that the clamping force of the electrode lock 5 on the electrode is increased.

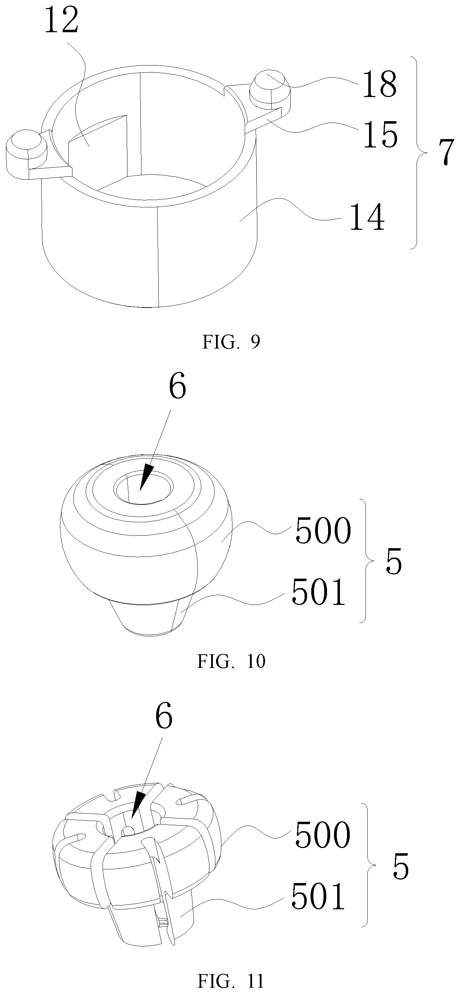

In some possible implementations, the electrode lock 5 includes a tightening portion 500 and a clamping portion 501 that communicate with each other. The tightening portion 500 and the clamping portion 501 are integrally connected to each other, the tightening portion 500 is at least partially disposed in the through hole 4 of the extension portion 3, and a lower-end part of the clamping portion 501 is located outside and below the through hole 4, as shown in FIG. 10 which is a structural view of a first electrode lock in the cranial hole electrode securing device according to an embodiment of the application of the present invention. For the structure of the first electrode lock 5, the material of the electrode lock 5 may be silica gel. The silica gel can reduce the possibility of the damage caused by an excessive clamping force of the electrode lock 5 on the electrode. Further, the tightening portion 500 has a universal ball joint structure, and the clamping portion 501 is a conical frustum structure whose large end is connected to the tightening portion 500. The tightening portion 500 with the preceding structure causes the electrode lock 5 to be adaptable to the electrode at an oblique implantation angle. The displacement of the electrode caused by the deviation of the angle of the electrode is reduced, and a more flexible path plan is also provided. The structure of a second electrode lock 5 is shown in FIG. 11 which is a structural view of the second electrode lock in the cranial hole electrode securing device according to an embodiment of the application of the present invention. For the structure of the second electrode lock 5, the material of the electrode lock 5 may be plastic. In this case, the tightening portion 500 and the clamping portion 501 are integrally connected to each other to form a collet structure. The collet structure is adaptable to a relatively large electrode, thereby increasing the use range of the cranial hole electrode securing device. It is to be noted here that when the sleeve 14 is rotated to squeeze the extension portion 3, the extension portion 3 compresses the tightening portion 500, thereby causing the tightening portion 500 to drive the clamping portion 501 to contract and clamp the electrode, which is very convenient and reduces the difficulty of the operation.

In some possible implementations, the material of the locking assembly 7 is metal. In some possible implementations, the material of the locking assembly 7 is a titanium alloy or stainless steel. The locking assembly 7 made of metal is less likely to deform and fail, is more durable, and has a longer service life.

In some possible implementations, as shown in FIG. 1 and FIGS. 4 to 7, the ring portion 2 is provided with multiple securing slots 21 for securing the electrode, and the multiple securing slots 21 are evenly distributed about the central axis of the cranial ring 1. In some possible implementations, the number of securing slots 21 is not specifically limited. Thus, a wire of the electrode is led out at multiple positions, thereby increasing the range of applicability of the cranial hole electrode securing device.

Although the embodiments of the present application have been shown and described above, it is to be understood that the preceding embodiments are exemplary and should not be construed as limiting the present application. Without departing from the principle and spirit of the present application, those of ordinary skill in the art may make any changes, modifications, substitutions, and variations on the preceding embodiments within the scope of the present application. All of these changes should fall within the scope of the claims of the present application.

## Claims

1. A cranial hole electrode securing device, comprising:
a cranial ring configured to be secured on a cranium, wherein the cranial ring comprises a ring portion and an extension portion, the extension portion is connected to a bottom end of the ring portion, and the cranial ring is provided with a through hole penetrating through the ring portion and the extension portion;
an electrode lock configured to clamp an electrode, wherein at least part of the electrode lock is disposed in a through hole of the extension portion, and the electrode lock is provided with a clamping opening configured for the electrode to penetrate through; and
a locking assembly movably disposed at the bottom end of the ring portion and sleeved on an outer side of the extension portion;
wherein when the locking assembly is moved from a first position to a second position, the locking assembly applies an acting force to the extension portion, and an opening at a bottom end of the through hole contracts and compresses the electrode lock, causing a positional lock of the electrode lock and/or causing the clamping opening to contract to clamp the electrode; and when the locking assembly is moved from the second position to the first position, the acting force applied by the locking assembly on the extension portion disappears or decreases, the opening at the bottom end of the through hole does not compress the electrode lock, causing the positional lock of the electrode lock to be released or causing the constraint of the electrode lock on the electrode to be released.

2. The cranial hole electrode securing device according to claim 1, wherein a sidewall of the extension portion is provided with at least one contraction groove extending upward from a bottom of the extension portion and penetrating through an inside and an outside of the extension portion, the extension portion is evenly divided into at least two connected parts by the at least one contraction groove, an outer sidewall of each of at least one part of the extension portion is provided with at least one first protrusion, and an inner sidewall of the locking assembly is provided with at least one second protrusion corresponding in position to the at least one first protrusion;
when the locking assembly is rotated such that each first protrusion abuts against a respective second protrusion, the locking assembly applies an acting force on each first protrusion of the extension portion through a respective second protrusion, parts of the extension portion with the at least one first protrusion converge toward a central axis of the extension portion and compress the electrode lock to contract the clamping opening to clamp the electrode; and
when the locking assembly is rotated such that each first protrusion is away from a respective second protrusion, each part of the extension portion does not compress the electrode lock.

3. The cranial hole electrode securing device according to claim 2, wherein the ring portion is provided with at least one lug slot, the locking assembly comprises a sleeve and at least one extension lug, the at least one extension lug is connected to an edge of a top of the sleeve and extends away from a central axis of the sleeve, the at least one extension lug corresponds in position to and matches in size with the at least one lug slot, and a bottom of each of the at least one extension lug is configured to be in contact with the cranium; and
when the sleeve is rotated from the first position to the second position, each of the at least one extension lug is rotated from one side of a respective lug slot to another side of the respective lug slot, each of the at least one first protrusion is caused to abut against a respective second protrusion, and parts of the extension portion with the at least one first protrusion converge toward the central axis of the extension portion to compress the electrode lock to clamp the electrode.

4. The cranial hole electrode securing device according to claim 3, further comprising a cranial hole cover, wherein the ring portion is provided with at least one snapping slot, and the cranial hole cover is engaged with the cranial ring through the at least one snapping slot and is configured to seal the electrode lock in the through hole; and
when the cranial hole cover snaps into the at least one snapping slot, a top surface of the cranial hole cover is not higher than a top surface of the ring portion.

5. The cranial hole electrode securing device according to claim 4, wherein the locking assembly further comprises limiting columns, wherein each of the limiting columns is connected to a side of a respective extension lug facing the cranial hole cover, and the cranial hole cover is provided with limiting holes which correspond in position to and match in size with the limiting columns; and
when the cranial hole cover is connected to the cranial ring, each of the limiting columns is inserted into a respective one of the limiting holes and restrains the sleeve from rotating from the second position to the first position.

6. The cranial hole electrode securing device according to claim 3, wherein an outer diameter of the sleeve is not greater than 8 mm, a height of the sleeve is not greater than a depth of the cranial hole, the ring portion is configured to fit snugly against an outer surface of the cranium, and a height of the ring portion is not greater than 2 mm.

7. The cranial hole electrode securing device according to claim 1, wherein an inner wall of the through hole in the extension portion is provided with a ring-shaped slot structure extending outward and transitioning smoothly to a rest part of the inner wall of the through hole, a part of the electrode lock located in the through hole of the extension portion has a universal ball joint structure which snugly fits against the inner wall of the through hole in the extension portion, a part of the electrode lock extending below the through hole tapers from top to bottom, and a size of an opening at a lower end of the clamping opening is not greater than a size of an opening at an upper end of the clamping opening.

8. The cranial hole electrode securing device according to claim 1, wherein the electrode lock comprises a tightening portion and a clamping portion that communicate with each other, the tightening portion and the clamping portion are integrally connected to each other, the tightening portion is at least partially disposed in the through hole of the extension portion, and a lower-end part of the clamping portion is located outside and below the through hole.

9. The cranial hole electrode securing device according to claim 8, wherein a material of the electrode lock is silica gel, the tightening portion has a universal ball joint structure, and the clamping portion is a conical frustum structure whose large end is connected to the tightening portion.

10. The cranial hole electrode securing device according to claim 8, wherein a material of the electrode lock is plastic, and the tightening portion and the clamping portion are integrally connected to each other to form a collet structure.

11. The cranial hole electrode securing device according to claim 1, wherein a material of the locking assembly is metal.

12. The cranial hole electrode securing device according to claim 11, wherein the material of the locking assembly is a titanium alloy or stainless steel.

13. The cranial hole electrode securing device according to claim 1, wherein the ring portion is provided with at least two threaded holes, and each of the at least two threaded holes is configured to be penetrated through by a screw to secure the cranial ring on the cranium.

14. The cranial hole electrode securing device according to claim 1, wherein the ring portion is provided with a plurality of securing slots configured to secure the electrode, and the plurality of securing slots are evenly distributed about a central axis of the cranial ring.
